(19) Europäisches Patentamt
European Patent Office
Office européen des brevets

(11) Veröffentlichungsnummer: **0 593 866 A1**

# (12) EUROPÄISCHE PATENTANMELDUNG

(21) Anmeldenummer: **93112715.3**

(22) Anmeldetag: **09.08.93**

(51) Int. Cl.[5]: **C08G 59/10**, C08G 59/28, C07C 215/18, C07C 219/06, G02F 1/35

(30) Priorität: **21.08.92 DE 4227798**

(43) Veröffentlichungstag der Anmeldung: **27.04.94 Patentblatt 94/17**

(84) Benannte Vertragsstaaten: **BE CH DE FR GB IT LI NL**

(71) Anmelder: **Siemens Aktiengesellschaft**
**Wittelsbacherplatz 2**
**D-80333 München(DE)**

(72) Erfinder: **Nordmann, Jens, Dr.**
**Oberer Grenzweg 40A**
**D-91077 Neunkirchen(DE)**
Erfinder: **Pühl, Rainer, Dipl.-Ing. (FH)**
**Neustadter Strasse 30**
**D-96450 Coburg(DE)**

## (54) Vernetzte Epoxidharze mit nichtlinear-optischen Eigenschaften.

(57) Zur Verwendung in nichtlinear-optischen Medien dienen - in vernetzter Form - Epoxidharze folgender Struktur:

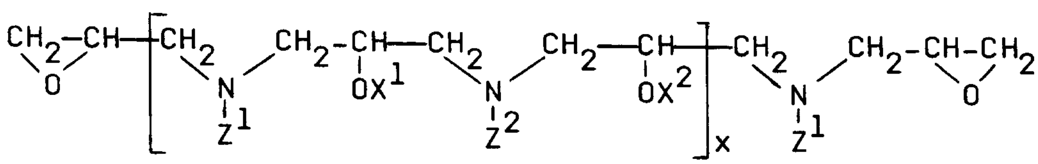
,

wobei $Z^1$ und $Z^2$ NLO-Chromophore, $X^1$ und $X^2$ H oder -CO-R-COOH sind und x = 1-20 ist.

Als NLO-Chromophore Z werden 4'-Nitrostilben oder 4'-Nitroazobenzol bevorzugt.

Dargestellt werden die Epoxidharze durch Umsetzung von mindestens 2 Mol eines Bisglycidylamins $Z^1-NR_2$ mit 1 Mol eines Amins $Z^2-NH_2$.

Die Vernetzung kann dann thermisch oder photochemisch erfolgen.

EP 0 593 866 A1

Die Erfindung betrifft vernetzte Epoxidharze sowie deren Herstellung und Verwendung.

Die nichtlineare Optik befaßt sich mit der Wechselwirkung des elektromagnetischen Feldes einer Lichtwelle mit einem Medium, in welchem sich die Lichtwelle ausbreitet, sowie mit dem damit verbundenen Entstehen neuer Felder mit veränderten Eigenschaften. Tritt nämlich das elektromagnetische Feld mit dem aus einem Molekül oder aus vielen Molekülen bestehenden Medium in Wechselwirkung, so polarisiert dieses Feld die Moleküle.

Die Polarisation, die durch ein lokales elektrisches Feld in einem Molekül induziert wird, kann - entsprechend Gleichung (1) - als Potenzreihe der elektrischen Feldstärke dargestellt werden:

$$P = \alpha.E + \beta.E^2 + \gamma.E^3 + ... \quad (1);$$

P ist dabei die induzierte Polarisation und E das induzierte lokale elektrische Feld, und $\alpha$, $\beta$ und $\gamma$ stellen die Polarisierbarkeit erster, zweiter und dritter Ordnung dar.

Auf makroskopischer Ebene gilt - entsprechend Gleichung (2) - für die durch ein äußeres elektrisches Feld in einem aus mehreren Molekülen bestehenden Medium induzierte Polarisation eine ähnliche Beziehung:

$$P = \epsilon_o(\chi^{(1)}.E + \chi^{(2)}.E^2 + \chi^{(3)}.E^3 + ...) \quad (2);$$

P ist dabei wiederum die induzierte Polarisation und E das induzierte lokale elektrische Feld, $\epsilon_o$ ist die Dielektrizitätskonstante, und $\chi^{(1)}$, $\chi^{(2)}$ und $\chi^{(3)}$ stellen die dielektrische Suszeptibilität erster, zweiter und dritter Ordnung dar.

Die dielektrischen Suszeptibilitäten in Gleichung (2) haben eine ähnliche Bedeutung wie die molekularen Koeffizienten in Gleichung (1): Sie sind Materialkonstanten, die von der Molekülstruktur und der Frequenz und im allgemeinen auch von der Temperatur abhängen. Materialien mit einer dielektrischen Suszeptibilität zweiter Ordnung eignen sich zur Frequenzverdoppelung; dies ist die Umwandlung von Licht der Frequenz $\omega$ in solches der Frequenz $2\omega$. Ein weiterer nichtlinear-optischer Effekt zweiter Ordnung ist der lineare elektrooptische Effekt (Pockels-Effekt); er resultiert aus der Änderung des Brechungsindex des optischen Mediums bei angelegtem elektrischen Feld. Die optische Gleichrichtung sowie die Summen- und Differenzfrequenzmischung sind weitere Beispiele für nichtlinear-optische Effekte zweiter Ordnung. Einsatzgebiete für Materialien der genannten Art sind beispielsweise elektrooptische Schalter sowie Bereiche der Informationsverarbeitung und der integrierten Optik, wie optische chip-to-chip-Verbindungen, wave-guiding in elektrooptischen Schichten, Mach-Zehnder-Interferometer und die optische Signalverarbeitung in der Sensorik.

Materialien mit einer dielektrischen Suszeptibilität dritter Ordnung eignen sich zur Frequenzverdreifachung der eingestrahlten Lichtwelle. Weitere Effekte dritter Ordnung sind die optische Bistabilität und die Phasenkonjugation. Konkrete Anwendungsbeispiele sind die holographische Datenverarbeitung und der rein optische Schalter zum Aufbau rein optischer Computer.

Zur Erzielung eines ausreichenden nichtlinear-optischen Effektes zweiter Ordnung muß die dielektrische Suszeptibilität zweiter Ordnung $\chi^{(2)}$ größer sein als $10^{-9}$ elektrostatische Einheiten (esu); dies bedeutet, daß die Hyperpolarisierbarkeit $\beta$ größer sein muß als $10^{-30}$ esu. Eine weitere fundamentale Voraussetzung zur Erzielung eines nichtlinear-optischen Effektes zweiter Ordnung ist die nicht-zentrosymmetrische Orientierung der Moleküle im nichtlinear-optischen Medium; andernfalls wird nämlich $\chi^{(2)} = 0$. Dies kann, wenn nicht - wie bei kristallinen Materialien - durch die Kristallstruktur vorgegeben, durch eine Orientierung der molekularen Dipole erreicht werden. So sind die größten Werte von $\chi^{(2)}$ für ein nichtlinearoptisches Medium durch Orientierung der molekularen Dipole in elektrischen Feldern erreicht worden.

Anorganische Materialien, wie Lithiumniobat ($LiNbO_3$) und Kaliumdihydrogenphosphat ($KH_2PO_4$), zeigen nichtlinearoptische Eigenschaften. Auch Halbleitermaterialien, wie Galliumarsenid (GaAs), Galliumphosphid (GaP) und Indiumantimonid (InSb), weisen nichtlinear-optische Eigenschaften auf. Neben dem Vorteil eines hohen elektrooptischen Koeffizienten zweiter Ordnung haben anorganische Materialien der genannten Art jedoch einige entscheidende Nachteile. So ist die Verarbeitung dieser Materialien technisch sehr aufwendig, da einzelne Prozeßschritte zeitaufwendig sind und mit höchster Genauigkeit durchgeführt werden müssen (siehe dazu: C. Flytzanis und J.L. Oudar "Nonlinear Optics: Materials and Devices", Springer-Verlag (1986), Seiten 2 bis 30). Diese Materialien sind ferner für solche elektrooptische Bauteile ungeeignet, die bei hohen Modulationsfrequenzen arbeiten. Bedingt durch die intrinsisch vorhandenen hohen Dielektrizitätskonstanten treten bei hohen Frequenzen (oberhalb einiger GHz) nämlich so hohe dielektrische Verluste auf, daß ein Arbeiten bei diesen Frequenzen unmöglich ist (siehe dazu: "J. Opt. Soc. Am. B", Vol. 6 (1989), Seiten 685 bis 692).

Es ist bekannt, daß organische und polymere Materialien mit ausgedehnten $\pi$-Elektronensystemen, die mit Elektronendonatoren und -akzeptoren substituiert sind, in nichtlinear-optischen Medien Verwendung finden können (siehe dazu: R.A. Hann und D. Bloor "Organic Materials for Nonlinear Optics", The Royal Society of Chemistry (1989), Seiten 382 bis 389 und 404 bis 411). Einkristalle auf organischer Basis weisen - im Vergleich zu $LiNbO_3$ - einen hohen elektrooptischen Koeffizienten zweiter Ordnung und eine gute photochemische Stabilität auf; auch die erforderliche hohe Orientierung der nichtlinear-optischen Moleküle ist bereits gegeben. Einige wesentliche Kriterien sprechen jedoch gegen eine technische Nutzung dieser Materialklasse. So wird für die Herstellung der Einkristalle, und zwar sowohl aus einer Lösung als auch aus der Schmelze, eine Zeit von 14 bis 30 Tagen benötigt (siehe dazu: D.S. Chemla und J. Zyss "Nonlinear Optical Properties of Organic Molecules and Crystals", Academic Press, Inc. (1987), Vol. 1, Seiten 297 bis 356); der Herstellungsprozeß wird somit einer technischen Fertigung nicht gerecht. Außerdem liegt der Schmelzpunkt der Kristalle im Mittel bei 100 °C, so daß ein Arbeitstemperaturbereich bis 90 °C nicht zu realisieren sein dürfte. Ferner sind organische Kristalle nicht strukturierbar und ihre lateralen Abmessungen sind gegenwärtig noch zu gering, um einen Aufbau als elektrooptisches Bauelement zu ermöglichen.

Als Werkstoffe für Anwendungen der nichtlinearen Optik in den Bereichen der Informationsübertragung und der integrierten Optik finden in neuerer Zeit polymere Materialien eine zunehmende Bedeutung. Diese polymeren Materialien lassen sich in der Weise herstellen, daß an eine über die Glasübergangstemperatur erwärmte Probe ein äußeres elektrisches Feld angelegt wird; dies führt zu einer Orientierung der nichtlinear-optischen Moleküle. Nach dem Abkühlen der Polymerprobe unter die Glasübergangstemperatur, bei angelegtem elektrischen Feld, wird ein anisotropes und somit nicht-zentrosymmetrisches Polymer erhalten, das dielektrische Suszeptibilitäten zweiter Ordnung aufweist.

Nichtlinear-optische Verbindungen, die in Polymeren gelöst oder in Polymere eindiffundiert sind, lassen sich zu dünnen Schichten verarbeiten, wie dies von der integrierten Optik gefordert wird (siehe dazu: "Macromolecules", Vol. 15 (1982), Seiten 1385 bis 1389; "Appl. Phys. Lett.", Vol. 49 (1986), Seiten 248 bis 250; "Electron. Lett.", Vol. 23 (1987), Seiten 700 und 701). Nachteilig wirkt sich hierbei jedoch die geringe Löslichkeit der niedermolekularen Verbindungen, ihre ungenügende Verteilung in den Polymeren, die Migration der aktiven Moleküle aus der Polymermatrix und der Verlust der nicht-zentrosymmetrischen Orientierung der aktiven Molekülspezies schon bei Raumtemperatur aus.

Als nichtlinear-optische Verbindungen sind auch Polymere mit kovalent gebundenen nichtlinear-optischen Molekülbausteinen bekannt, die gleichzeitig flüssigkristallinen Charakter besitzen (siehe dazu: EP-OS 0 231 770 und EP-OS 0 262 680). Diese Materialien weisen zwar die vorstehend genannten Nachteile nicht auf, sie sind aber im gegenwärtigen Entwicklungsstadium für Anwendungen in der Elektro- oder integrierten Optik nicht geeignet, da sich hierbei optische Verluste > 20 dB/cm ergeben, die durch die inhärente Domänenstreuung hervorgerufen werden. Ferner wurde bereits über Untersuchungen an amorphen nichtlinear-optischen Polymeren berichtet (siehe: "Macromolecules", Vol. 21 (1988), Seiten 2899 bis 2901).

Sowohl bei flüssigkristallinen als auch bei amorphen Polymeren mit kovalent gebundenen nichtlinear-optischen Moleküleinheiten kann eine sehr viel höhere Konzentration an diesen Moleküleinheiten verwirklicht werden. Ein Spacer entkoppelt dabei die molekulare Beweglichkeit der nichtlinear-optischen Einheiten von der Polymerkette; gleichzeitig nimmt jedoch die Glasübergangstemperatur drastisch ab. Damit ist aber bei Gebrauchstemperaturen im Bereich der Glasübergangstemperatur der Polymere der Verlust der molekularen Orientierung der nichtlinear-optischen Moleküleinheiten und der Verlust der nichtlinear-optischen Aktivität zu erwarten.

Aufgabe der Erfindung ist es, das Angebot an Polymeren für nichtlinear-optische Medien zu erweitern und dabei insbesondere Polymere zur Verfügung zu stellen, die eine hohe Glasübergangstemperatur bei gleichzeitig erhöhter nichtlinear-optischer Aktivität aufweisen, welche auch hohen technischen Anforderungen genügt.

Dies wird erfindungsgemäß durch vernetzte Epoxidharze erreicht, die aus niedermolekularen Epoxidharzen folgender Struktur erhalten werden:

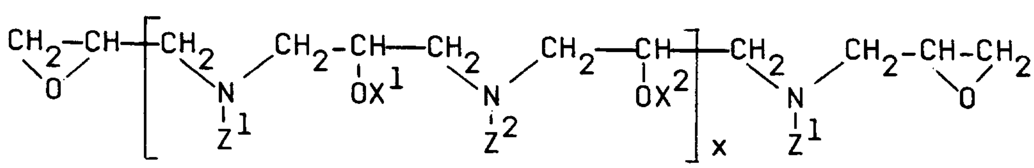 ,

wobei folgendes gilt:

x = 1 bis 20;

$X^1$ und $X^2$ = H oder -CO-R-COOH, wobei R eine aliphatische, cycloaliphatische oder aromatische Gruppierung ist;

$Z^1$ und $Z^2$ ist jeweils ein mit einem Elektronenakzeptor (A) substituiertes konjugiertes $\pi$-Elektronensystem (E) der Struktur -E-A, wobei folgendes gilt:

E = 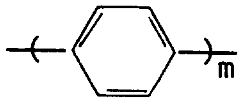 , 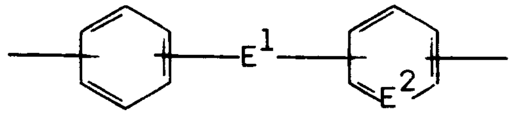

oder

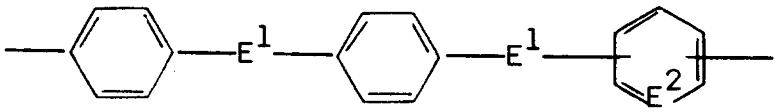 ,

mit

- m = 1 bis 3,
- $E^1$ = $-(CH=CH)_n-$, -N=N-, -CH=N-, -N=CH- oder -C≡C-, mit n = 1 bis 3, und
- $E^2$ = CH oder N;
- A = -NO, $-NO_2$, -CN, $-CF_3$, $-SO_2OR^1$, $-SO_2NR^2_2$ ,

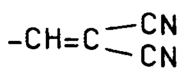

oder

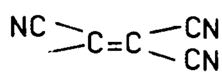 ,

mit

$R^1$ und $R^2$ = Wasserstoff, Alkyl, Fluoralkyl, Alkenyl, Aryl oder Heteroaryl.

Vorzugsweise gilt folgendes:

- x = 1 bis 10,
- $X^1$ = $X^2$ = H oder $-CO-(CH_2)_2-COOH$,
- Z = -E-A mit A = $-NO_2$ oder

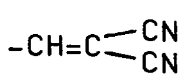 ,

und

E = 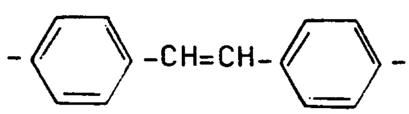

oder

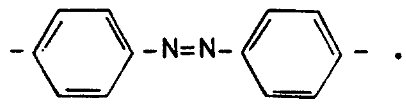

Die konjugierten π-Elektronensysteme (E) können auf der Elektronenakzeptorseite - zusätzlich zu A - mit wenigstens einem weiteren Elektronenakzeptor substituiert sein. Diese Substituenten sollten so ausgewählt werden, daß die Summe der Hammetkonstanten σ der zusätzlichen Substituenten den Wert des vorhandenen Substituenten (A) nicht überschreitet.

Die Erfindung besteht somit in vernetzten Epoxidharzen der vorstehend genannten Art. Ferner besteht die Erfindung in der Verwendung dieser vernetzten Epoxidharze in orientierter Form für nichtlinear-optische Medien bzw. in nichtlinear-optischen Polymeren in Form vernetzter Epoxidharze der vorstehenden Struktur.

Es wurde nämlich überraschenderweise gefunden, daß vernetzte Epoxidharze der beschriebenen Art mit kovalent gebundenen nichtlinear-optischen Moleküleinheiten einerseits die vorstehend genannten Nachteile nicht aufweisen, andererseits aber die bekannt guten polymerspezifischen Eigenschaften besitzen, wie Verarbeitbarkeit zu dünnen Schichten im µm-Bereich, hohe Konzentration an nichtlinear-optischen Moleküleinheiten, niedrige optische Dämpfung und technisch ausreichende Glasübergangstemperatur. Vorteilhaft ist bei den erfindungsgemäßen Polymeren ferner, daß die Möglichkeit einer Strukturierung der nichtlinear-optischen Polymerschicht zu Wellenleiterstrukturen durch Vernetzungsreaktionen besteht, wie sie bei unvernetzten Polymeren nicht möglich sind.

Aus der EP-OS 0 430 143 sind vernetzte Epoxidharze mit nichtlinear-optischen Eigenschaften bekannt, bei denen die NLO-Chromophore sowohl in der Haupt- als auch in der Seitenkette gebunden sein können. Nachteilig wirkt sich dabei der niedrige Gehalt von ≦ 10 Gew.-% an NLO-Chromophor im vernetzten Produkt aus, da größere Mengen eines aliphatischen und/oder aromatischen Diamins als zusätzliches Vernetzungsmittel eingesetzt werden müssen.

Aus der EP-OS 0 477 667 sind Epoxidharze bekannt, die kovalent gebundene NLO-Chromophore enthalten und in vernetzter Form für nichtlinear-optische Medien verwendet werden. Diese Epoxidharze sind aus NLO-inaktiven Diglycidylverbindungen und NLO-aktiven Monoaminen aufgebaut. Im Gegensatz dazu dienen zum Aufbau der Epoxidharze nach der Erfindung NLO-aktive Diglycidylverbindungen. Dies hat den Vorteil, daß dadurch eine wesentliche Steigerung der nichtlinear-optischen Aktivität erreicht wird.

Die erfindungsgemäßen vernetzten Epoxidharze werden in der Weise hergestellt, daß ein glycidylfunktionalisiertes NLO-Chromophor der Struktur

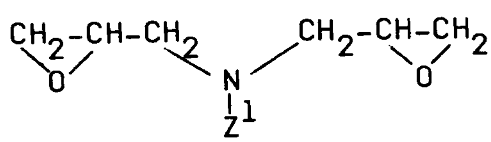

(1)

mit einem NLO-Chromophor der Struktur

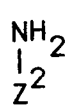

(2)

im Molverhältnis ≧ 2 zur Reaktion gebracht und das dabei erhaltene niedermolekulare Epoxidharz vernetzt wird. Das glycidylfunktionalisierte NLO-Chromophor (1) ist vorzugsweise 4-(N,N-Diglycidylamino)-4'-nitrostilben oder 4-(N,N-Diglycidylamino)-4'-nitroazobenzol, das NLO-Chromophor (2) vorzugsweise 4-Amino-4'-nitrostilben oder 4-Amino-4'-nitroazobenzol.

Zur Herstellung der vernetzten Polymere werden somit aus den NLO-Chromophoren zunächst die Epoxidharze synthetisiert, die - nachdem sie zu dünnen Schichten mit nichtlinear-optischen Eigenschaften im µm-Bereich verarbeitet wurden - in den vernetzten Zustand überführt werden. Bei der Synthese der Epoxidharze oder vor deren Vernetzung können an sich bekannte NLO-Chromophor-unmodifizierte Epoxidharze in Konzentrationen zwischen 5 und 75 Masse-%, vorzugsweise zwischen 5 und 25 Masse-%,

zugesetzt werden. So lassen sich nichtlinear-optische Medien mit hoher Konzentration an nichtlinear-optischen Moleküleinheiten, niedriger optischer Dämpfung und technisch ausreichender Glasübergangstemperatur herstellen. Besonders vorteilhaft ist auch, daß die Vernetzung der Epoxidharze zu den vernetzten Polymeren mit nichtlinear-optischen Eigenschaften vor, während oder nach der erforderlichen Orientierung der vorhandenen nichtlinear-optischen Moleküleinheiten durchgeführt werden kann.

Die Herstellung nichtlinear-optischer Polymere durch Vernetzung auf chemischem Weg ist bereits bekannt (siehe: "J. Appl. Phys.", Vol. 66 (1989), Seiten 3241 bis 3247). Dazu werden durch Umsetzung von Bisphenol A-diglycidylether mit 4-Nitro-1.2-phenylendiamin zunächst lösliche Prepolymere hergestellt, die dann durch Erhitzen in unlösliche vernetzte Polymere überführt werden. Auf entsprechende Weise können auch nichtlinear-optische Polymere aus N,N-Diglycidyl-4-nitroanilin und N-(2-Aminophenyl)-4-nitroanilin hergestellt werden (siehe: "Appl. Phys. Lett.", Vol. 56 (1990), Seiten 2610 bis 2612). Aus "J. Opt. Soc. Am. B", Vol. 7 (1990), Seiten 1239 bis 1250, sind ferner unvernetzte elektrooptische Polymerfilme bekannt, die durch Umsetzung von 4-Nitroanilin mit Bisphenol A-diglycidylether erhalten werden. Diese Polymermaterialien weisen jedoch den Nachteil auf, daß die Orientierung der nichtlinear-optischen Moleküleinheiten durch Coronapolung während der Vernetzung der Polymere durchgeführt wird. Erfahrungsgemäß ist auf diese Weise aber keine optimale Orientierung der Moleküleinheiten möglich, da die verwendete Polungsmethode zu einer verminderten Orientierungsstabilität der NLO-Einheiten führt (siehe dazu: "Macromolecules", Vol. 23 (1990), Seiten 1891 bis 1894).

Die Herstellung der Epoxidharze nach der Erfindung sowie die Synthese der Vorstufen erfolgt nach an sich bekannten Verfahren (vgl. dazu die Ausführungsbeispiele). Epoxidharze, bei denen $X^1$ und/oder $X^2$ die Bedeutung -CO-R-COOH hat, werden durch Umsetzung der entsprechenden Verbindungen mit OH-Gruppen mit Carbonsäureanhydriden hergestellt (siehe dazu: DD-PS 248 598 sowie "Makromol. Chem.", Bd. 190 (1989), Seiten 2673 bis 2681). Geeignete Carbonsäureanhydride sind insbesondere Bernsteinsäureanhydrid, Phthalsäureanhydrid, Tetrahydro-, Hexahydro-, Methyltetrahydro-und Endomethylentetrahydrophthalsäureanhydrid sowie Pyromellithsäure-, Trimellithsäure und Benzophenontetracarbonsäureanhydrid.

Die Vernetzung der Epoxidharze nach der Erfindung kann entweder thermisch oder photochemisch erfolgen. Die thermische Vernetzung wird durch Erhitzen auf eine Temperatur bewirkt, die oberhalb der Glasübergangstemperatur des vernetzten Endproduktes, d.h. des Polymers, liegt, vorzugsweise ca. 15 °C darüber. Zur thermischen Vernetzung können den Epoxidharzen vorteilhaft imidazolhaltige Initiatoren in einer Konzentration zwischen 0,5 und 10 Masse-%, vorzugsweise zwischen 1 und 5 Masse-%, zugesetzt werden.

Die photochemische Vernetzung der Epoxidharze erfolgt durch Licht kürzerer Wellenlänge, vorzugsweise durch Licht im UV-Bereich (290 bis 390 nm). Zur Auslösung der photochemischen Vernetzung werden der Reaktionsmischung Initiatoren zugesetzt, die unter dem Einfluß von Licht Lewis- oder Brönstedsäuren freisetzen; derartige Verbindungen sind an sich bekannt (siehe beispielsweise: "J. Macromol. Sci., Rev. Macromol. Chem.", Vol. C21 (1982), Seiten 187 bis 273). Insbesondere haben sich Aryldiazonium-, Diaryljodonium- und Triarylsulfoniumsalze, die als Anion Tetrafluoroborat, Hexafluorophosphat, Hexafluoroarsenat oder Hexafluoroantimonat aufweisen, sowie Aren-Eisen-Salze als geeignet erwiesen.

Zur Verbesserung der Oberflächenbeschaffenheit, der Verarbeitbarkeit und/oder der Verträglichkeit mit anderen Polymeren können den Epoxidharzen - je nach Einsatzzweck - Verarbeitungshilfsmittel zugesetzt werden. Dies sind beispielsweise Thixotropiermittel, Verlaufmittel, Weichmacher, Netzmittel, Gleitmittel und Bindemittel.

Die Epoxidharze nach der Erfindung werden in gelöster oder flüssiger Form, gegebenenfalls zusammen mit vernetzungswirksamen Verbindungen oder Initiatoren, durch Aufschleudern, Tauchen, Bedrucken oder Bestreichen auf ein Substrat aufgebracht. Auf diese Weise erhält man eine nichtlinear-optische Anordnung, wobei die Epoxidharze vorzugsweise nach der Vernetzungreaktion in elektrischen Feldern dipolar ausgerichtet, d.h. orientiert werden. Es werden Polymermaterialien mit ausgezeichneten nichtlinear-optischen Eigenschaften und - bedingt durch die Vernetzung - erhöhter Orientierungsstabilität und somit erhöhter Langzeitstabilität, auch bei erhöhten Gebrauchstemperaturen, erhalten. Diese Polymere gelangen in nichtlinear-optischen Anordnungen zum Einsatz, insbesondere in optischen Bauelementen.

Anhand von Ausführungsbeispielen soll die Erfindung noch näher erläutert werden.

Beispiel 1

Herstellung von N,N-Bis(3-chlor-2-hydroxypropyl)-anilin

1,0 mol Anilin und 2,4 mol Epichlorhydrin werden in 375 ml Ethanol 16 h unter Rückfluß erhitzt. Dann wird das Lösungsmittel im Vakuum entfernt und der Rückstand in n-Hexan eingetropft. Die dabei erhaltenen Kristalle werden abfiltriert und getrocknet (Ausbeute: 75 %); Fp.: 60 °C.

Beispiel 2

Herstellung von 4-(N,N-Diglycidylamino)-4'-nitroazobenzol

0,25 mol 4-Nitroanilin werden in 150 ml 16 %iger wäßriger Salzssäure verrührt und bei 0 bis 5 °C mit 0,25 mol Natriumnitrit in 100 ml Wasser diazotiert. Die Diazoniumsalzlösung wird filtriert, bei 5 bis 10 °C zu einer Lösung von 0,25 mol N,N-Bis(3-chlor-2-hydroxypropyl)-anilin in 250 ml 5 %iger wäßriger Salzsäure getropft und dann 1 h gerührt. Die dabei erhaltene Suspension wird mit 25 %iger waßriger Ammoniaklösung neutralisiert, anschließend werden 1000 ml 50 %ige wäßrige Natronlauge zugetropft, und dann wird 1 h gerührt. Der Niederschlag wird abgesaugt und neutral gewaschen. Nachfolgend wird das Rohprodukt getrocknet und an Kieselgel mit Dichlormethan/Tetrahydrofuran als Elutionsmittel chromatographiert (Ausbeute: 34 %); Fp.: 135 bis 136 °C.

Beispiel 3

Herstellung von 4,4'-Dinitrostilben

3,5 mol p-Nitrobenzylchlorid werden in 2300 ml Ethanol in der Wärme gelöst, und dazu wird eine Lösung von 3,8 mol Kaliumhydroxid in 1100 ml wäßrigem Ethanol (70:30) getropft. Nach beendeter Zugabe wird im Eisbad auf 0 °C abgekühlt, der Niederschlag abfiltriert und mit heißem Wasser und warmem wäßrigen Alkohol (50:50) gewaschen. Dann wird im Vakuum bei 75 °C getrocknet (Ausbeute: 83 %).

Beispiel 4

Herstellung von 4-Amino-4'-nitrostilben

0,34 mol 4,4'-Dinitrostilben werden in 1800 ml siedendem Pyridin gelöst. Innerhalb von 30 min werden zu dieser Lösung 540 ml einer 1-molaren Lösung von $Na_2S/S$ in Wasser/Pyridin (9:1) zugetropft. Anschließend wird die Mischung 15 min unter Rückfluß erhitzt, und dann werden 2500 ml Wasser zugesetzt. Nach dem Abkühlen im Eiswasserbad wird der Niederschlag abfiltriert, mit Wasser und Methanol gewaschen und aus Chlorbenzol umkristallisiert (Ausbeute: 60 %).

Beispiel 5

Synthese eines Epoxidharzes aus 4-(N,N-Diglycidylamino)-4'-nitroazobenzol und 4-Amino-4'-nitrostilben

8,46 mmol 4-(N,N-Diglycidylamino)-4'-nitroazobenzol und 4,23 mmol 4-Amino-4'-nitrostilben werden zusammen mit 0,85 mmol Bisphenol A-diglycidylether bei 165 °C aufgeschmolzen, homogenisiert und 2 h bei dieser Temperatur unter Rühren erhitzt. Das nach dem Erkalten erhaltene Umsetzungsprodukt weist eine Glasübergangstemperatur $T_G$ von 99 °C auf (Ausbeute: 100 %).

Beispiel 6

Härtung des Epoxidharzes aus 4-(N,N-Diglycidylamino)-4'-nitroazobenzol und 4-Amino-4'-nitrostilben

Zur Härtung, d.h. Vernetzung, wird das Umsetzungsprodukt nach Beispiel 5, gelöst in einem geeigneten Lösungsmittel unter Zusatz von 1,5 Masse-% eines Imidazolinitiators, auf eine Unterlage aus Glas aufgeschleudert und 3 h bei 150 °C gehärtet. Das dabei erhaltene Produkt weist eine Glasübergangstemperatur $T_G$ von 160 °C auf.

Beispiel 7

Herstellung von 4-(N,N-Diglycidylamino)-4'-nitrostilben

0,1 mol 4-Amino-4'-nitrostilben, 1,0 mol Epichlorhydrin und 0,05 mol Essigsäure werden 48 h auf 75 °C erhitzt; anschließend werden die flüchtigen Bestandteile im Vakuum abdestilliert. Der Destillationsrückstand wird in 250 ml Tetrahydrofuran gelöst und bei 50 °C zu 160 ml einer 50 %igen wäßrigen Natriumhydroxidlösung getropft. Dann wird 30 min gerührt, abgekühlt und die organische Phase abgetrennt. Die organische Phase wird nachfolgend mit 1000 ml Wasser versetzt, der ausgefallene Niederschlag wird abfiltriert, neutral gewaschen, getrocknet und aus Isopropanol umkristallisiert (Ausbeute: 70 %).

Beispiel 8

Synthese eines Epoxidharzes aus 4-(N,N-Diglycidylamino)-4'-nitrostilben und 4-Amino-4'-nitrostilben

8,46 mmol 4-(N,N-Diglycidylamino)-4'-nitrostilben und 4,23 mmol 4-Amino-4'-nitrostilben werden zusammen mit 0,85 mmol Bisphenol A-diglycidylether bei 130 °C aufgeschmolzen, homogenisiert und 2 h bei dieser Temperatur unter Rühren erhitzt. Das nach dem Erkalten erhaltene Umsetzungsprodukt weist eine Glasübergangstemperatur $T_G$ von 73 °C auf (Ausbeute: 100 %).

Beispiel 9

Härtung des Epoxidharzes aus 4-(N,N-Diglycidylamino)-4'-nitrostilben und 4-Amino-4'-nitrostilben

Zur Härtung, d.h. Vernetzung, wird das Umsetzungsprodukt nach Beispiel 8, gelöst in einem geeigneten Lösungsmittel unter Zusatz von 3,0 Masse-% eines Imidazolinitiators, auf eine Unterlage aus Glas aufgeschleudert und 3 h bei 150 °C gehärtet. Das dabei erhaltene Produkt weist eine Glasübergangstemperatur $T_G$ von 145 °C auf.

Beispiel 10

Für die elektrooptischen Untersuchungen werden die erfindungsgemäßen Epoxidharze, gegebenenfalls zusammen mit vernetzungswirksamen Verbindungen, in einem geeigneten Lösungsmittel auf ITO-beschichtetes Glas (ITO = Indium-Zinn-Oxid) durch Spin-coating aufgebracht; die Schichtdicke von in dieser Weise hergestellten Filmen betragt üblicherweise 3 bis 6 µm. Für das elektrische Polen, zur Erzielung einer hohen nicht-zentrosymmetrischen Orientierung, wird auf den Film (aus dem Epoxidharz) eine Goldelektrode aufgesputtert; die Gegenelektrode ist dabei die lichtdurchlässige ITO-Schicht. Nach dem Erwärmen der Probe bis in den Glasübergangstemperaturbereich wird eine Gleichspannung angelegt, wobei die erforderliche Spannungserhöhung auf das Orientierungsverhalten der nichtlinear-optischen Moleküleinheiten abgestimmt wird, um elektrische Durchschläge und somit eine Zerstörung des Films zu vermeiden. Nach Erreichen einer Polungsfeldstärke von 50 bis 100 V/µm genügt eine Polungsdauer von 15 min zur Orientierung der nichtlinear-optischen Moleküleinheiten. Anschließend wird die Probe vernetzt, und zwar thermisch (wie in den Beispielen 6 und 9) oder photochemisch, und dann wird die Probe - mit konstant anliegendem elektrischen Feld - bis auf Raumtemperatur abgekühlt, womit die Orientierung fixiert wird.

Die elektrooptische Untersuchung der Polymerproben erfolgt durch interferometrische Messung eines schräg eingestrahlten Laserstrahls nach Einfachreflexion an der Goldelektrode. Der dazu erforderliche Meßaufbau und die Meßauswertung sind bekannt (siehe beispielsweise: "Appl. Phys. Lett.", Vol. 56 (1990), Seiten 1734 bis 1736). Der elektrooptische Koeffizient $r_{33}$ des Polymers nach Beispiel 6, der auf eine Polungsfeldstärke von 100 V/µm bezogen ist, beträgt 70 pm/V, derjenige des Polymers nach Beispiel 9, bezogen auf die gleiche Polungsfeldstärke, 60 pm/V.

**Patentansprüche**

**1.** Vernetzte Epoxidharze, hergestellt aus niedermolekularen Epoxidharzen der Struktur

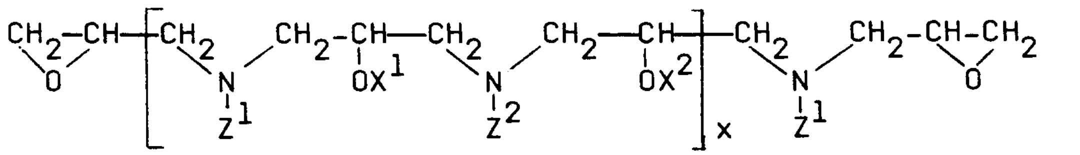

wobei folgendes gilt:

x = 1 bis 20;

$X^1$ und $X^2$ = H oder -CO-R-COOH, wobei R eine aliphatische, cycloaliphatische oder aromatische Gruppierung ist;

$Z^1$ und $Z^2$ ist jeweils ein mit einem Elektronenakzeptor (A) substituiertes konjugiertes $\pi$-Elektronensystem (E) der Struktur -E-A, wobei folgendes gilt:

E = 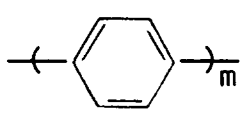 , 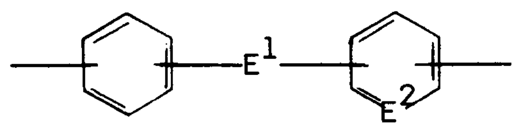

oder

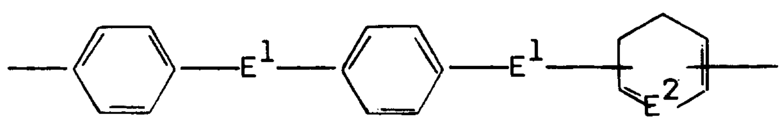 ,

mit

m = 1 bis 3,

$E^1$ = $-(CH=CH)_n-$, -N=N-, -CH=N-, -N=CH- oder -C≡C-, mit n = 1 bis 3, und

$E^2$ = CH oder N;

A = -NO, $-NO_2$, -CN, $-CF_3$, $-SO_2OR^1$, $-SO_2NR^2_2$ ,

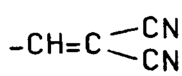

oder

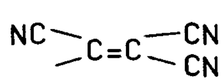 ,

mit

$R^1$ und $R^2$ = Wasserstoff, Alkyl, Fluoralkyl, Alkenyl, Aryl oder Heteroaryl.

2. Epoxidharze nach Anspruch 1, **dadurch gekennzeichnet,** daß folgendes gilt:

x = 1 bis 10,

$X^1$ = $X^2$ = H oder $-CO-(CH_2)_2-COOH$,

Z = -E-A mit A = $-NO_2$ oder

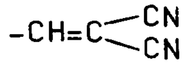 ,

und

E = 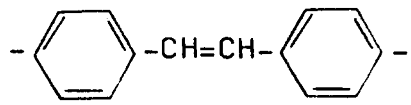

oder

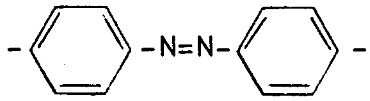 .

3. Verfahren zur Herstellung von vernetzten Epoxidharzen nach Anspruch 1 oder 2, **dadurch gekennzeichnet,** daß ein glycidylfunktionalisiertes NLO-Chromophor der Struktur

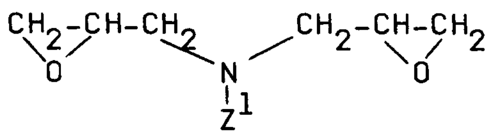 (1)

mit einem NLO-Chromophor der Struktur

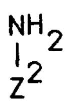 (2)

im Molverhältnis ≧ 2 zur Reaktion gebracht und das dabei erhaltene niedermolekulare Epoxidharz vernetzt wird.

4. Verfahren nach Anspruch 3, **dadurch gekennzeichnet,** daß als glycidylfunktionalisiertes NLO-Chromophor der Struktur (1) 4-(N,N-Diglycidylamino)-4'-nitrostilben oder 4-(N,N-Diglycidylamino)-4'-nitroazobenzol eingesetzt wird.

5. Verfahren nach Anspruch 3 oder 4, **dadurch gekennzeichnet,** daß als NLO-Chromophor der Struktur (2) 4-Amino-4'-nitrostilben oder 4-Amino-4'-nitroazobenzol eingesetzt wird.

6. Verfahren nach einem der Ansprüche 3 bis 5, **dadurch gekennzeichnet,** daß die Vernetzung thermisch oder photochemisch erfolgt.

7. Verfahren nach Anspruch 6, **dadurch gekennzeichnet,** daß die thermische Vernetzung in Gegenwart eines imidazolhaltigen Initiators in einer Konzentration von 0,5 bis 10 Masse-% durchgeführt wird.

8. Verfahren nach einem oder mehreren der Ansprüche 3 bis 7, **dadurch gekennzeichnet,** daß NLO-chromophorfreie Epoxidharze in einer Konzentration zwischen 5 und 75 Masse-% zugesetzt werden.

9. Verfahren nach Anspruch 8, **dadurch gekennzeichnet,** daß die Konzentration der NLO-chromophorfreien Epoxidharze zwischen 5 und 25 Masse-% beträgt.

**10.** Verwendung der vernetzten Epoxidharze nach Anspruch 1 oder 2 in orientierter Form für nichtlinear-optische Medien.

Europäisches Patentamt

# EUROPÄISCHER RECHERCHENBERICHT

Nummer der Anmeldung

EP 93 11 2715

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | EP-A-0 477 667 (SIEMENS AG)<br>* Ansprüche 1-3; Seite 3, Zeile 51 - Seite 6, Zeile 34 *<br>--- | 1-6,8-10 | C 08 G 59/10<br>C 08 G 59/28<br>C 07 C 215/18<br>C 07 C 219/06<br>G 02 F 1/35 |
| Y | POLYMERS FOR ADVANCED TECHNOLOGIES, Band 3, Nr. 5, August 1992, Seiten 211-217, Chichester, Sussex, GB; R. ZENTEL et al.: "Polymeric liquid crystals: structural basis for ferroelectric and nonlinear optical properties"<br>* Seiten 213,214 *<br>--- | 1-6,8-10 | |
| Y | SPIE - The International Society for Optical Engineering Proceedings, Band 1147, August 1989, Seiten 74-83; B. RECK et al.: "Crosslinked epoxy polymers with large and stable nonlinear optical susceptibilities"<br>* Seiten 74-78 *<br>--- | 1-6,8-10 | |
| | | | RECHERCHIERTE SACHGEBIETE (Int. Cl.5) |
| D,A | EP-A-0 430 143 (THE DOW CHEMICAL COMPANY)<br>* Ansprüche 1-25; Seite 5, Zeile 16 - Seite 8, Zeile 27 *<br>----- | 1-10 | C 08 G<br>C 07 C<br>G 02 F |

Der vorliegende Recherchenbericht wurde für alle Patentansprüche erstellt

| Recherchenort | Abschlußdatum der Recherche | Prüfer |
|---|---|---|
| BERLIN | 29-12-1993 | KRISCHE D |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)